# EUROPEAN PATENT APPLICATION

(11) **EP 2 671 597 A1**
(43) Date of publication of application: **11.12.2013**
(21) Application number: 12171160.0
(22) Date of filing: 07.06.2012
(51) Int. Cl.: A61K 47/48, A61P 35/00, C07K 7/00

(54) **Chimeric constructs between glioma-homing peptide and cell-penetrating peptides, gHoPe2**

(71) Applicant: Cepep III AB, 104 30 Stockholm (SE)
(72) Inventor: Kurrikoff, Kaido, 50090 Tartu (EE); Eriste, Elo, 50090 Tartu (EE); Oskolkov, Nikita, 50705 Tartu (EE); Suhorutsenko, Julia, 50090 Tartu (EE); Langel, Ülo, 51013 Tartu (EE); Howl, John, Wolverhampton, West Midlands WV1 1LY (GB); Jones, Sarah, Wolverhampton, West Midlands WV1 1LY (GB)
(74) Representative: Sarap, Margus

(57) **Abstract**

The current invention is based on a unique targeting entity (the homing peptide, which we designate as ''gHo''). gHo is a peptide sequence that has specific properties (glioma targeting), the sequence is characterized within the current invention, by using specific methodology. The novel gHo peptide is further utilized in a delivery system, which, in addition to gHo, consists of the delivery entity (a CPP) and a cargo (the effector molecule, such as chemotherapeutic drug). Therefore, the delivery system itself is also novel, because it combines the transport activity of the CPP and the targeting activity of the gHo.

## Description

### Technical Field

The present invention generally relates to the field of cancer, molecular medicine and drug delivery. Specifically, the present invention relates to targeted drug delivery for augmenting chemotherapy against glioblastomas. In specific embodiment, the present invention relates to diagnostic labeling of the glioblastoma tumor tissue(s).

### Background Art

Tumors represent the most common cause of death in humans. Despite that much effort has been invested on developing new treatment strategies and anti-cancer medicines, especially for breast cancers, there is still large number of clinical cases when the patient cannot be wholly recovered and tumor removed with current pharmaceutical tools and/or other oncologic measures. The current invention relates to chemotherapy, a major sub-field of clinical anti-cancer intervention.

Currently available anti-cancer chemotreatment is generally quite effective, but has serious drawbacks that are addressed with our invention. The drawbacks are: (i) currently available chemotherapy is not effective for brain tumors and (ii) chemotherapy is not specific towards cancer cells because treatment is not targeted enough.

Chemotherapy is not effective for brain tumors. The main reason for that is that the drug is cleared (removed from the body) before it reaches brain parenchyma. Drug delivery to the most distant tumor cells, which are behind the blood-brain-barrier, is limited by insufficient penetration of systemically administered drugs. Our hypothesis for the invention was that by vectorizing the chemotherapeutic drug, using a CPP for this, we are able to improve penetration to the brain and thus increase the potency of the original drug.

Chemotherapy is not specific. As chemotherapy lacks specificity towards tumor cells, it is highly toxic to normal tissues. Within the current invention, a novel tumor-binding entity is used in conjunction with the chemotherapeutic drug, in order to increase the specificity towards cancer (glioma) cells. The benefits of increasing specificity of the drug are twofold: (i) the safety of the treatment increases, because the toxicity towards normal tissues decreases and (ii) higher doses can be used, which increases the anticancer efficacy of the treatment.

***Delivery mechanism***

The current invention is also related to the field of drug delivery, which is a discipline that explicitly, and in controlled manner aims to modulate pharmacokinetics and pharmacodynamics of specific drug molecules, by means of delivery vectors. In general, the drug molecule is vectorized, or bound to the delivery vector, and in combination with specific delivery vector formulation protocol, yielding a delivery system.

One way of achieving drug delivery is using cell-penetrating peptides (CPPs). This is a relatively newer class of transport vectors that have received increasing attention in last years. These cationic and/or amphipathic peptides are usually less than 30 amino acids in length and have the ability to rapidly translocate into most of the mammalian cells. The usefulness of these peptides arises from their ability to carry along various cargo molecules that can not reach into the cells by themselves. These cargo molecules may include oligonucleotides, peptides, proteins, plasmids, liposomes, and nanoparticles both *in vitro* and *in vivo* [1]. The cargo may be reversibly or irreversibly linked to the CPP. Thus, CPPs have opened a new avenue in drug delivery, allowing otherwise impermeable, therapeutic agents to enter cells and induce biological responses.

The 18-amino acid long peptide pVEC is one of the well characterized CPP [2]. It is suitable for drug delivery research target because of its high cell penetrating efficacy and low toxicity [2-4]. pVEC is derived from the murine vascular endothelial-cadherin (VE-cadherin) protein which main function is to mediate physical contact between adjacent cells by homophilic dimerization.

***Targeting mechanism***

The general function of the CPPs enables nonspecific drug delivery. However, in the field of cancer research the aim is to achieve transfer of a cargo (for example chemotherapeutic agent) to only a special subpopulation of all the cells (i.e, only into the tumor cells). Thus, a control mechanism for the transport is needed. One way to accomplish this is by using target-specific antibodies. For example, knowing that specific types of cells (in this case, tumor cells are cells of interest) express certain molecule in their cell membrane, antibodies can be produced against that antigen and conjugated to the CPP (5-6). Another way of achieving a controlled transport is to use an inactive transport vector that is activated near the target of interest (7-9). Yet another way of achieving CPP selectivity is to use homing peptides - peptides that bind to specific target molecules of a (tumor) cell - as a conjugated domain of the CPP (for example, (10) has utilized this strategy).

Thus, the present invention addresses the need for targeted drug delivery to brain tumors. There is a great demand for the targeting delivery mechanism that could be applied to already existing medicines. In addition, there is a need for delivery of a variety of therapeutic and diagnostic molecules over normally not permeable blood-brain barrier and cell membrane.

### Summary of invention

The present invention provides composition and methods for composing transport vector with cell-selective properties, for delivering different types of cargo molecules and substances into target cells. The compositions herein exploit the ability of cell-penetrating peptides to translocate cargo molecules across blood-brain barrier and cell membrane. The inventors have described a targeting mechanism, a homing peptide that binds to brain tumor cells. The inventors have found that when the homing peptide is covalently linked to the cell-penetrating peptide, selective transport of a cargo can be achieved. The cargo can be any molecule that can be covalently linked to the CPP.

Therefore the current invention is based on a unique targeting entity (the homing peptide, which we designate as "gHo"). gHo is a peptide sequence that has specific properties (glioma targeting), the sequence is characterized within the current invention, by using specific methodology.

The novel gHo peptide is further utilized in a delivery system, which, in addition to gHo, consists of the delivery entity (a CPP) and a cargo (the effector molecule, such as chemotherapeutic drug). Therefore, the delivery system itself is also novel, because it combines the transport activity of the CPP and the targeting activity of the gHo.

An embodiment of the invention includes a chemotherapeutic drug as a cargo. For example, the drug can be 6-thioguanine, chlorambucil, doxorubicin, epirubicin, or any other chemotherapeutic drug molecule that can be covalently linked to a peptide.

An embodiment of the invention includes a proapototic peptide as a cargo. The cargo can also be any molecule that affects cell cycle or has cytotoxic properties and that can be covalently linked to a peptide.

An embodiment of the invention includes a diagnostic agent as a cargo. The diagnostic agent can be a contrast agent for the MRI, a radioactive agent, a radiopaque agent, a fluorophore, a fluorescence-quenching system, a marker, a marker enzyme (such as horse-radish peroxidase, beta-galactosidase or other enzyme suitable for tagging the cell), or any other molecule that can be used as a diagnostic entity and that can be covalently linked to a peptide.

### Brief description of drawings

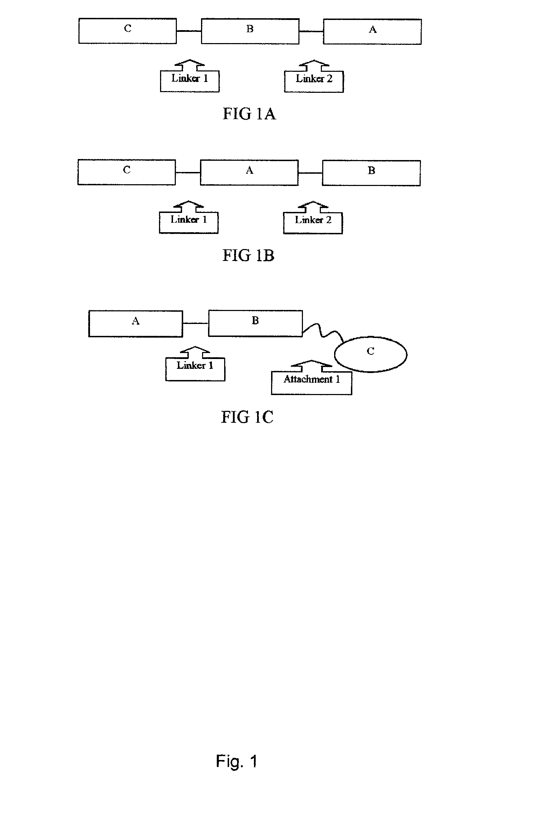

Figure 1A-1C. General scheme that represents the core of the current invention and methods of application. A represents the unique peptide sequence that has targeting properties, and/or which adds targeting properties to the whole delivery system. B represents the carrier system, which, in (a) and (b) is covalently linked to A and C. In case of (c) the component B is covalently to A, but the compoetnt C is associated in different way, such as when component B is a liposome, micelle, dendrimer, or nanoparticle. C is a cargo. A, B, and C are linked to or associated with each other via specific linkers (Linker 1, Linker 2), or via other association (Attachment 1).

Figure 2. Association of the targeting peptide gHo with glioma cells (U87) and with HeLa and HEK cells. The peptide was applied at the concentration of 20 µM. gHo associated with U87 cells, but not with HEK and HeLa cells.

Figure 3. Cellular internalization of the cell-penetrating peptide-based targeting compounds, labelled with FAM, in human glioma cells (U87). All peptides were applied at a concentration of 10 µM. The free targeting peptide (FAM-gHo) showed no uptake, while FAM-pVEC, FAM-gHoPe2 and FAM-gHoPe3 exhibited cellular uptake of varying degrees.

Figure 4. Cellular internalization of the cell-penetrating peptide-based targeting compounds, labelled with FAM, in human glioma cells, measured by FACS. The uptake of FAM-pVEC and FAM-gHoPe2 was highest.

Figure 5. Localization of cell-penetrating peptide-based targeting compounds, labelled with FAM, in subcutaneous human glioma (U87) tumours. The peptides were administered i.v. 3 h before tissue collection. Cryosections from the subcutaneous tumours, as well as from brain, kidney and liver were counterstained with DAPI and visualised by FAM-fluorescent microscopy. FAM-gHoPe2 localizes in tumours. Scalebar 100 µm.

Figure 6. Coronal section of a mouse brain with human glioma tumour (U87) in the right striatum, Bregma +1 mm. (A) H&E stained hemisphere of the brain and glioma, reconstructed from single microscopic images. The animals received an i.v. injection of FAM-labelled gHoPe2 3h before tissue collection. (B) FAM-fluorescence image from the same location as A (reconstructed from single fluorescence microscopic images), indicating global FAM localization in the tumour area.

Figure 7. Localization of cell-penetrating peptide-based targeting compounds, labelled with FAM, in intracranial human gliomas and intact brain, as well as in liver and kidney. FAM-gHoPe2 localizes in tumours. Scale 100 µm.

Figure 8. Localization of cell-penetrating peptide-based targeting compounds, labelled with FAM, in muscle, lung, heart, skin and spleen of the animals with intracranial tumours. No uptake was detected in any of the tissues. Scale 100 µm.

Figure 9 and Figure 10. Evaluation of the toxicity of FAM-labelled chimeric peptides in U87 cells. (A) MTS cell viability assay for the treatment with gHoPe2, pVEC and gHoPe3. The peptides did not induce any toxic effects. (B) LDH leakage assay for the treatment with gHoPe2, pVEC, gHoPe3 and gHo in U87 cells. The peptides did not induce any significant membrane leakage as compared to 0.2% of Triton.

Figure 11. Scheme of the doxorubicin-gHoPe2 conjugate (dox-gHoPe2).

Figure 12. The effect of doxorubicin, dox-gHoPe2 and FAM-gHoPe2 in the viability of U87 cells according to the MTS assay. The constructs were incubated at different concentrations for 24h. Cells in untreated cultures were defined as having 100% viability.

Figure 13. U87 subcutaneous tumour growth dynamics after treatment with either free doxorubicin (i.v. 1 mg/kg twice a week) or with an equivalent molar dose of dox-gHoPe2 (treatment days indicated with arrows). Star represents Tukey post-hoc comparison of treatment groups after significant repeated-measures ANOVA. *p<.05.

### Description of embodiments

The present invention provides composition and methods for targeted delivery of a range of cargo molecules and substances into specific cells. In the described invention, a targeting mechanism is described, which can be used to specifically reach, bind, affect or internalize certain type of cells.

In addition, an example of the application of the targeting mechanism is characterized, where the targeting mechanism is coupled to a mechanism of cellular internalization, to provide the means of reaching cell interior. Finally, a third entity is coupled to the targeting mechanism. This provides means for the intracellular effector role, for example, cell death or cell labeling. The novel targeting mechanism is selectively binding to human brain glioma cells, and is used a vehicle for delivering the substance of interest only into the tumor cells. The desired property of the described delivery vector is that it has a flexible design, allowing (i) coupling and subsequent selective transport of different compounds, depending on the intended purpose of the resulting vector and (ii) coupling of different types of carriers for providing cellular internalization.

The invention is thus comprised of three components, linked by defined chemical linkers, but the order of the three components is not strictly defined, as schematically depicted in Figure 1A - 1C.

**The component A**

The component A in Figure 1A -1C is the targeting peptide. The sequence of the targeting peptide (or "homing peptide", as sometimes referred to in the current text) is given in Table 1 (see SEQ No. 1). We subsequently refer to this peptide as "gHo" (an acronym of words representing glioma-homing). The component A is attached or linked to other components of the delivery vector. The general aim of the component A is providing targeting function for achieving delivery of component C specifically into glioma cells.

**The component B**

The component B in Figure 1A - 1C is a carrier element, which combines all components under a unifying delivery and targeting system. It acts as a vehicle for the component C and attachment basis for the component A. Depending on which specific carrier is used, B may also act as an enhancer for cellular entry, enhancer for blood-brain barrier penetration, enhancer for endosomal escape, protector against blood serum degradation, modifier for the pharmacokinetic liberation of the cargo, inhibitor of the clearance from the organism after in vivo administration, and it can be linked to components of A and/or C either covalently or associated with A and/or C noncovalently.

The component B can be any cell-penetrating peptide (CPP), covalently linked to A and C. The component B can be positioned differently in the final delivery molecule, for example, as depicted in Figure 1A and Figure1B. The component B can also be other peptide, which do not strictly fall under the category of cell-penetrating peptides, but which still functions to assist with the delivery (such as polylysine, protamine, albumin etc). The component A can also be covalently linked to other types of carrier, which facilitate delivery and/or release of cargo in the cells, with the aim of using component A as a targeting moiety.

The component B can be other type of carrier, which associates with the component C noncovalently, and uses the component A as a targeting moiety, as depicted in Figure 1C. In this case, the component B can be for example a liposome, micelle, dendrimer, cyclodextrin, or other types of similar carrier systems that fall under the classification of polyplexes or lipoplexes. This also includes carriers that involve modifications of the polyplexes or lipoplexes, such as PEG-modified liposomes.

**The component C**

Component C is a cargo that is delivered in or onto the cell, and which provides the intracellular effector function of the delivery vector. C can be a clinically approved chemotherapeutic drug, chemotherapeutic agent, other apoptotic, cytostatic or cytotoxic entity, as long as it can be covalently conjugated to the component A or component B, or otherwise associated with component B, as depicted in Figure 1A - 1C.

The component C can also be an imaging or diagnostic agent, providing a function of labeling the target cells.

Linker

The components A, B and C are linked to or associated with each other, comprising a unifying delivery system.

In case of covalent conjugation between two components, the linker can be a chemical linker that reduces steric hindrance, enhances chemical conjugation reaction process, and/or provides specific additional properties to the whole delivery vector, such as providing dissociation of the two linked components in the presence of specific environmental conditions (for example, at specific pH or in the presence of specific enzymes) or mediating a conformational change at the presence of specific environmental conditions, that mediate activation or inactivation of any function of the delivery vector.

In case of noncovalent association, the component C may be encapsulated within or in the interior of a liposome, micelle, dendrimer, polymer, polyplex or lipoplex. This is depicted in Figure 1C. In this case, the component A is attached to component B, providing targeting function.

The invention will now be further illustrated by the following description of embodiments, including short description of the drawings, materials and methods, examples including figures and figure legends as well as sequence listing, but it should be understood that the scope of the invention is not limited to any specifically mentioned embodiments or details.

***Materials and methods***

**Phage display**

U251 glioma cells were cultured in Dulbecco's Modified Eagle Medium (DMEM) with L-glutamine. Medium was supplemented with 10% (v/v) fetal bovine serum (FBS), penicillin (100 U/ml) and streptomycin (100 µg/ml). U251 cells were incubated with the M13 phage display library (Ph.D.-12™ New England Biolabs Inc.) at 3.75 x 10¹⁰ plaque forming units overnight at 4°C. Unbound phage were washed away by serial washing in culture medium containing 1% (w/v) bovine serum albumin (BSA). Bound phage were eluted by gentle agitation in 0.2M glycine-HCl (pH 2.2) containing 1 mg/ml BSA for 10 minutes and subsequently neutralized with 1 M Tris-Hcl (pH 9.1). Cell-specific phage were amplified in *E.Coli* and subjected to four rounds of panning according to the manufacturer's instructions (New England Biolabs). High affinity individual clones were identified by DNA sequencing (AltaBioscience, Birmingham, UK).

**Peptide synthesis**

Peptides (Table 1) were synthesized in a stepwise manner at a 0.1 mmol scale on an automated peptide synthesizer (Applied Biosystems™ model 433A) using Fmoc solid-phase peptide synthesis strategy [5]. Rink-amide MBHA (methylbenzylhydrylamine) resin (MultiSyntech GmbH, Germany) was used as a solid support to obtain C-terminally amidated peptides. All chemicals used in peptide synthesis were purchased from Sigma-Aldrich (Germany), MultiSyntech GmbH (Germany) and Scharlau (Spain).

For the *in vitro* and *in vivo* cellular uptake studies the peptides were N-terminally labelled with 5(6)-carboxyfluorescein (FAM) (Novabiochem) by treatment of peptidyl-resin with 5(6)-carboxyfluorescein (3 eq), N,N'-diisopropylcarbodiimide (3 eq), hydroxybenzotriazole (3 eq) and N,N-diisopropylethylamine (6 eq) in dimethyl sulfoxide (DMSO):dimethylformamide (1:1) overnight. The final cleavage was performed using a standard protocol (95% trifluoroacetic acid (TFA)/2.5% triisopropylsilane/2.5% water) for 3 h at room temperature.

Peptides were purified by using reversed phase HPLC fitted with a C18 column (Zorbax 300SB-C18, Agilent) and a gradient of 5-100% acetonitrile/water containing 0.1 % TFA for 45 min. The molecular weight of peptides was analyzed by MALDI-TOF mass-spectrometry (The Voyager-DE™ PRO Biospectrometry™ System) in positive linear mode using α-cyano-4-hydroxycinnamic acid as matrix (Sigma-Aldrich) and purity was more than 95% as determined by analytical HPLC.

For the preparation of doxorubicin conjugates, additional cysteine was added to the N-terminus, followed by the cleavage and purification of the peptides as described above. Doxorubicin was conjugated to peptides according to [6] with small alterations. Briefly, doxorubicin (2 mg) was dissolved in 500 µl of DMSO and then dispersed in a phosphate buffer solution (500 µl, pH 8.0) at a concentration of 2 mg/ml. After addition of 20 µl of triethylamine (TEA), 200 µl of succinimidyl-4-(N-maleimidomethyl)cyclohexane-1-carboxylate (SMCC, 10 mg/ml) was added. The mixture was incubated at a room temperature for 2h. After adjusting the pH of the mixture to 5.5 with 2M HCl, 300 µl of peptide solution (15 mg/ml) was added. The mixture was kept at a room temperature for an additional 2h. In order to remove free doxorubicin, SMCC and TEA, the reaction mixture was subjected to reversed-phase purification using a gradient of acetonitrile/water containing 0.1 % TFA (5-100% for 45 min). The identity and purity of the obtained conjugates were confirmed by using MALDI-TOF mass spectrometry and analytical HPLC, respectively.

**Cell culture**

The human embryonic kidney 293 cell line (HEK 293) and human glioblastoma-astrocytoma, epithelial-like cell line U87 MG (American Type Culture Collection via LGC, Sweden) and cervical cancer HeLa cells were cultured in Dulbecco's Modified Eagle Medium (DMEM) with L-glutamine. Medium was supplemented with 10% FBS, sodium pyruvate (1 mM), penicillin (100 U/ml), streptomycin (100 µg/ml) and 1% nonessential amino acids (further denoted as complete medium) (PAA Laboratories GmbH, Austria). Cell cultures were cultivated at 37°C in a humidified 5% CO₂ incubator.

**Flow cytometry**

U87, HEK and Hela cells (5×10⁴) were seeded 24 h prior to experiments into 24-well plates. Cells were incubated for 30 min in complete media after treatment with the peptide constructs. Thereafter, cells were washed four times with PBS and once with trypsin, suspended in ice-cold PBS and flow cytometry analysis was carried out with a BD LSR II flow cytometer (BD Biosciences, San Jose, CA, USA) equipped with a 488 nm argon laser and analyzed with FACSDiva software. The filter settings for excitation and emission were 480/530 nm bandpass (FL1) for FITC. Live cells were gated by forward/side scattering from a total of 10000 events.

**Confocal microscopy**

U87 cells (1×10⁴) were seeded in Lab-Tek® 8-well chamber slides to reach 50% confluence one day prior to experimentation. After 30 min of incubation with the peptide constructs in complete medium at 37°C, cells were washed four times with PBS, followed by the addition of trypan blue (0.1 %) in OptiMEM and examined using Olympus IX81 inverted microscope equipped with FluoView 1000 confocal system using a 60x water-immersion and 100x oil-immersion objectives and excitation at 488 nm (fluorescein). For the membrane association experiments the cells were incubated with FAM-labeled peptide for 30 min at 37°C in complete medium. After the incubation period, cells were washed once with PBS and re-suspended in fresh OptiMEM medium.

In vitro toxicity

Membrane integrity was evaluated using LDH leakage assay (SIGMA-ALDRICH™ St. Louise, MO, USA), which measures lactate dehydrogenase release. Briefly, 1×10⁴ cells were seeded 24h in 96-well plates before treatment with the peptides or peptide-drug conjugates, which was conducted in complete medium for 48h. Thereafter, LDH leakage was assessed according to the manufacturer's protocol. Untreated cells were defined as no leakage and 100% leakage was defined as total LDH release by lysing cells in 0.2% Triton X-100 in HKR buffer.

Long-term toxicity was evaluated using an MTS proliferation assay (Promega, Madison, WI, USA). The MTS assay measures the activity of mitochondrial dehydrogenases to convert tetrazolium salts into formazan. Briefly, 1×10⁴ cells were seeded 24h in 96-well plates before treatment with the peptides or peptide-drug conjugates, which was conducted in complete medium for 24h. Thereafter, MTS proliferation assay was used according to the manufacturer's protocol. Untreated cells were defined as 100% viable.

***Animal* experiments and postmortem tissue analysis**

Localization of the peptide vectors was assessed *postmortem* after injecting FAM-labeled peptides in homozygous female nude mice (C.Cg/AnNTac-Foxn1nu, Taconic), bearing tumor xenografts either intracranially or subcutaneously. 8×10⁵ U87 cells were resuspended in 100 µl (s.c tumor model) or in 5 µl (intracranial tumor model) of fresh DMEM medium, without serum and antibiotics. The xenografting was performed by implanting the cell suspension subcutaneously or intracranially into one of the striatums (stereotaxic coordinates A=+1, L=+2, V=+3.5). At the appearance of the first signs of tumor growth (for the subcutaneous tumors: tumor size of approx 300 mm³; for the intracranial tumors: the first sign is usually weight loss), mice were injected i.v. (via tail vein) with the peptide constructs. For the biodistribution, 100 µl of 200 µM FAM-labeled peptide suspension (in saline) was used. This represents a dose of 4 mg/kg for a mouse of 20 g. At 3h post-injection, the animals were anesthetized and intracardially perfused with 50 ml of PBS, followed by 50 ml of fixation solution (4% w/v paraformaldehyde in PBS, pH 7.4). Tissues were collected, postfixed for 2h in the same fixation solution, cryoprotected in sucrose (10% for 24h, followed by 30% for 1 week, at 4°C), snap frozen in 2-methylbutane (AppliChem) kept on dry ice. The tissues were cut on a cryostat with a thickness of 10-15 µm, counterstained with DAPI (AppliChem) and mounted using Vectashield (Vector Labs) mounting media.

The tumor treatments were performed using 1 mg/kg doxorubicin (369 µM, 100 µl, for an animal of 20 g) and an equivalent dose (8.3 mg/kg) of dox-gHoPe2 (369 µM, 100 µl) i.v. twice a week. The subcutaneous tumor dimensions were measured using calibre; tumor volume was calculated as (length × width²)/2.

A mixture of ketamine 75 mg/kg (Vetoquinol, Bioketan) and dexmedetomidine (Laboratorios SYVA, Dorbene) 1 mg/kg i.p in saline was used for the anesthesia. After surgery, the anesthesia was blocked using the α₂-adrenergic antagonist atipamezole hydrochloride (Antisedan), 1 mg/kg s.c.

All the animal procedures and experiments were approved by the Estonian laboratory animal ethics committee (approvals no 19, dated Sep 25^{th}, 2009, no 69 and 70, dated Feb 9^{th}, 2011).

**Statistics**

All figures are presented as mean ± SEM. The cell uptake data represent three independent experiments performed in duplicate. For toxicity measurements, a decrease in viability was considered significant at p <.001 using ANOVA Dunnett's multiple comparison test.

***Example 1_{:} characterization of the targeting peptide gHo***

Using in vitro phage display, we have characterized a novel peptide sequence which specifically binds glioma cells. The sequence of the targeting peptide (or "homing peptide", as sometimes referred to in the current text) is given in Table 1. We subsequently refer to this peptide as "gHo" (an acronym of words representing glioma-homing).

**Table 1**

| **Name** | **Sequence** |
|---|---|
| pVEC | LLIILRRRIRKQAHAHSK-NH₂ |
| gHo (SEQ No.1) | NHQQQNPHQPPM-NH₂ |
| FAM-pVEC | ^{a}LLIILRRRIRKQAHAHSK-NH₂ |
| FAM-gHo | ^{a}NHQQQNPHQPPM-NH₂ |
| FAM-pVEC-gHo | ^{a}LLIILRRRIRKQAHAHSK-NHQQQNPHQPPM-NH₂ |
| (FAM-gHoPe2) | |
| FAM-gHo-pVEC (FAM-gHoPe3) Dox-pVEC-gHo (Dox-gHoPe2) | ^{a}NHQQQNPHQPPM-LLIILRRRIRKQAHAHSK-NH₂ ^{b}XCLLIILRRRIRKQAHAHSK-NHQQQNPHQPPM-NH₂ |

*a - 5(6)-carboxyfluorescein*
*b - doxorubicin*
*X - succinimidyl-4-(N-maleimidomethyl) cyclohexane-1-carboxylate (SMCC)*

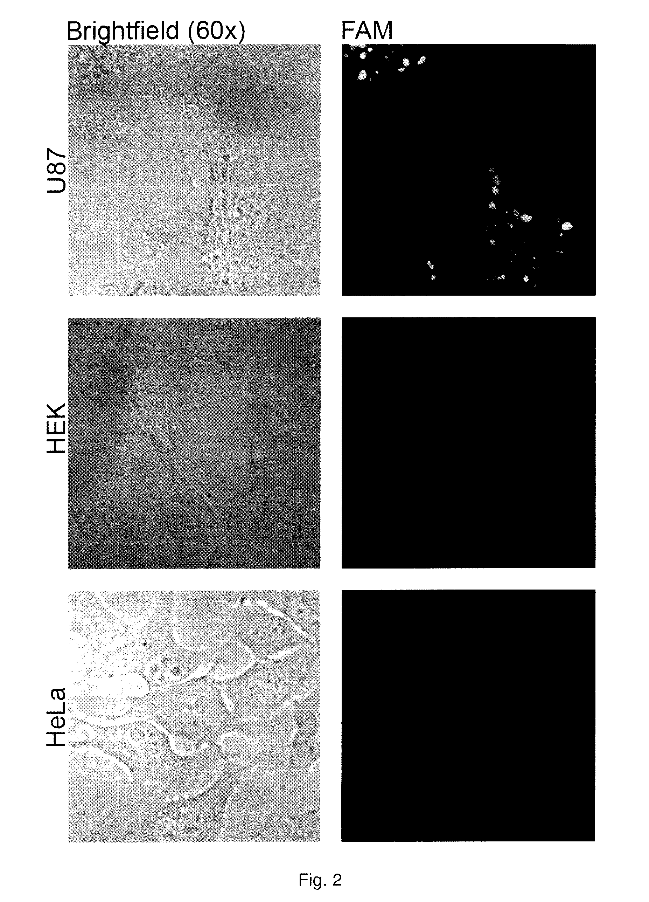

To confirm binding of the targeting peptide with glioma cells, gHo peptide was labeled with fluorescent marker FAM (see the composition in Table 1) and incubated with U87 glioma cells as well as with two non-neuronal cell lines, HeLa and HEK, for 30 minutes at the room temperature, followed by mild washing of the cells. Confocal microscopy confirmed association of gHo with U87 (Figure 2) at all the tested concentrations (1-20 µM of the peptide). gHo did not bind to HeLa and HEK cells even at the very high concentration of 20 µM.

***Example 2*: *construction of a targeted delivery vector, based on a cell-penetrating peptide***

The component B (Figure 1) can be a cell-penetrating peptide. In one embodiment, the cell-penetrating peptide may be a peptide pVEC [2]. The Linker 1 or Linker 2 in Figure 1 may be a chemical linker, which reduces steric hindrance or provides dissociation or cleavage of the molecule at specific environmental conditions. A series of pVEC-based delivery vectors are presented in Table 1, consisting of the tumor-homing peptide gHo, CPP, cargo, and a linker. For testing the delivery efficacy, a fluorescent marker was used as a test cargo (component C). For testing antitumour effect, doxorubicin was used as a component C.

***Example 3*: *in vitro cellular internalization of the targeted delivery vector***

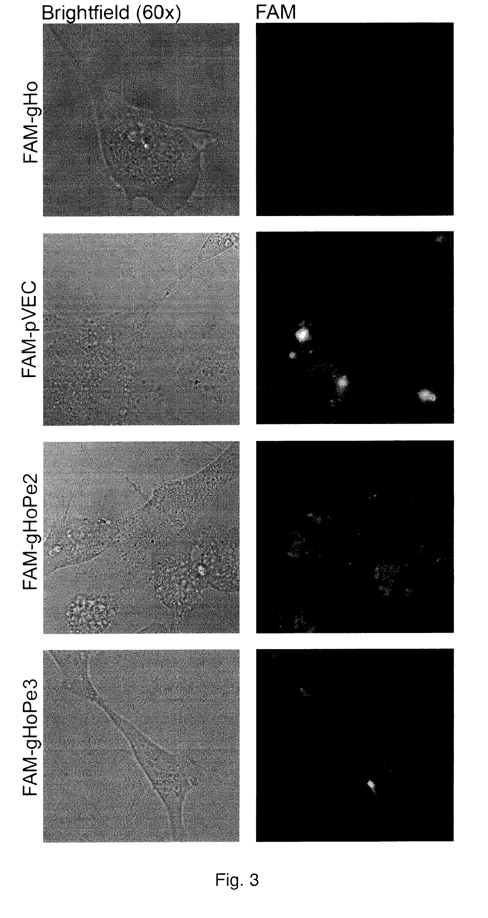

The cellular uptake of the chimeric peptides gHoPe2 and gHoPe3 in U87 cells, using confocal microscopy and flow cytometry is depicted in Figures 3 and 4, respectively.

Confocal microscopy showed that the free homing peptide gHo did not internalize in U87. As anticipated, the unmodified pVEC internalized very efficiently in the U87 cells. FAM-gHoPe2 showed similar internalization efficiency as pVEC while the FAM-gHoPe3 internalized, but to a lesser extent (Figure 3).

Quantitative comparison of the uptake efficacies (Figure 4) was in accordance with the microscopic observations. All the pVEC-based constructs showed high cellular uptake, while free gHo did not internalize. Statistical analysis showed ANOVA interaction between concentration and vector (F_{(15, 145)}=6.0, p<.001). The uptake of FAM-gHoPe2 significantly differed from the uptake values of FAM-gHoPe3 (main effect p<.01, Tukey *post hoc)* and FAM-gHo (p<.001).

***Example 4*: *biodistribution of the targeted delivery vector in vivo***

The biodistribution of FAM-labeled gHoPe2 was determined in two mouse tumour models. FAM-labeled gHo and gHoPe2 were injected 3h before tissue collection. The circulation time of 3 h was chosen from initial pilot experiments with 1 h, 3h, 6h and 24h circulation times, where the highest fluorescence in the tumor occurred at 3h.

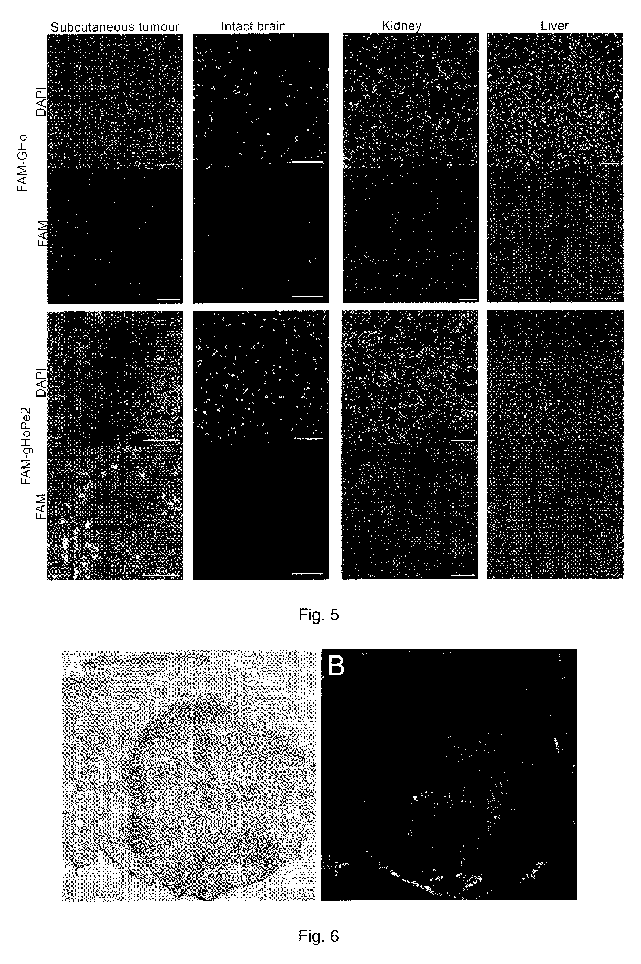

Firstly, a mouse model of subcutaneous U87 tumor was used. Intravenous (i.v) injection of FAM-labeled constructs in mice bearing subcutaneous tumors and subsequent postmortem histological analysis showed homing of the FAM-gHoPe2 to the tumor tissue (Figure 5). We detected no FAM-labeled gHo in tumor tissue and none of the peptides in the (intact) brain tissue, the kidney, and liver.

Secondly, biodistribution of FAM-labeled gHo and gHoPe2, as well as FAM-labeled pVEC and gHoPe3 was assessed in a mouse model of intracranial glioblastoma. To estimate the general distribution of gHoPe2 within the glioma-containing brain, the whole coronal section was reconstructed from single microscopic images. Cranial localization of gHoPe2 can be seen in the tumor area but not in the adjacent intact brain (Figure 6).

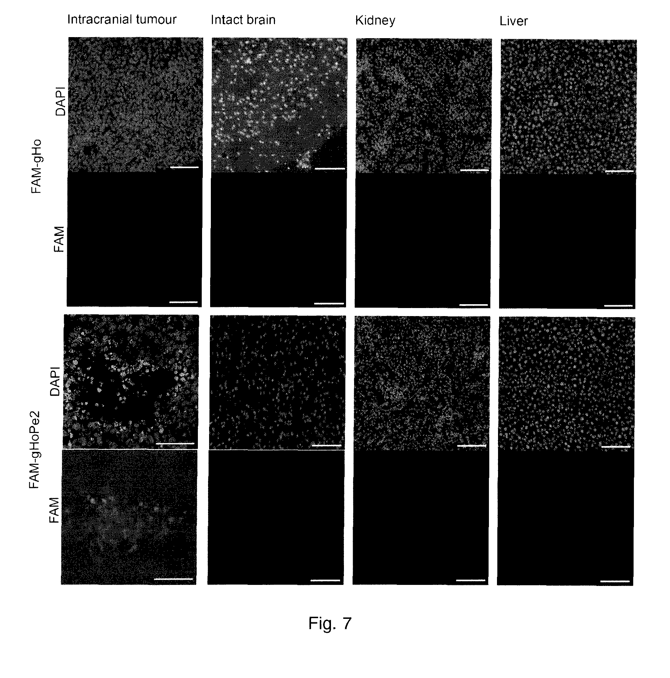

Closer examination of different tissues confirmed specific localization of gHoPe2 (Figure 7), but not gHo in tumor tissue. gHoPe2 was the only peptide present in the intracranial tumors, but not in adjacent intact brain, kidney and liver.

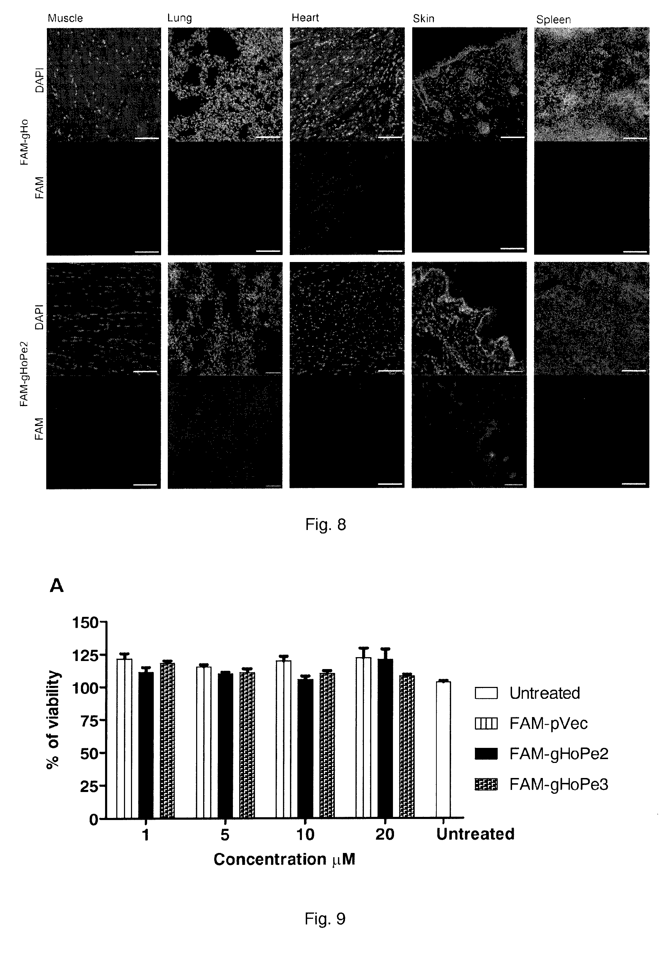

We also assessed the uptake of the constructs in other tissues. No uptake of gHoPe2 or gHo was seen in lungs, heart, spleen, muscle and skin (Figure 8).

***Example 5*: *cytostatic effect* in vitro**

The effect of the FAM-gHoPe2 was further assessed on cell viability. FAM-pVEC, FAM-gHoPe2 and FAM-gHoPe3 did not affect cell viability even at very high concentrations of 20 µM (Figure 9, Figure 10), measured using MTS cell viability assay and LDH leakage assay.

Component C in Figure 1 may be a chemotherapeutic molecule. Linker 1 or Linker 2 in Figure 1 may be succinimidyl-4-(N-maleimidomethyl) cyclohexane-1-carboxylate (SMCC). gHoPe2 was covalently conjugated with chemotherapeutic drug doxorubicin, using SMCC as a linker (Table 1, Figure 11) and its cytotoxic efficacy was assessed. Free doxorubicin had a clear effect on cell viability, with even low doses exerting quite large toxic effect (Figure 12). Dox-gHoPe2 exerted similar cytotoxic effects as the free drug.

***Example 6*: *antitumour effect* in vivo**

The efficacy of dox-gHoPe2 was assessed in the model of subcutaneous U87 tumors. The treatment was commenced when visible tumors (approx 300 mm³) had developed. The animals were randomized into three treatment groups; animals receiving either free doxorubicin (1 mg/kg (369 µM, 100 µl, for an animal of 20 g), dox-gHoPe2 (8.3 mg/kg (369 µM, 100 µl) i.v. twice a week, or no treatment (i.v. injection of saline). Tumor sizes were recorded twice a week. Repeated-measures ANOVA of the tumor growth dynamics (Figure 13) showed significant treatment main effect (F_{10, 65}=2.1, p<.05), the tumors in dox-gHoPe2 group being significantly smaller from the tumors in untreated group (Tukey *post-hoc* p<.05).

***Potential application of the invention***
1. Clinical treatment of brain tumors. Selective transport of antineoplastic agents (such as chemotherapeutic drugs) into the brain tumors after systemic injection of gHoPe2 coupled to the drug. This potential use is based on preclinical research results conducted animal models with human brain tumors.
2. Clinical diagnostic tool. Selective labeling of brain tumors after systemic injection of gHoPe2 coupled to a label.
3. Tumor research and drug discovery. In conjunction with novel drug candidates, development of new potential tumor treatment medicines and strategies, including compounds that lack the ability to cross blood-brain barrier and cell membrane.

Thus, the present invention can be used as a drug delivery vector during antineoplastic treatment (anti-cancer therapy). In this case, the cargo is clinically approved chemotherapeutic drug. The invention adds tumor-selective properties to the antineoplastic agents, allowing more focused delivery of the cytotoxic effect the chemotherapeutic drug. This allows physicians to use higher doses of medicines (and consequently achieve stronger clinical effect) at the same level of side effects. Alternatively, lower doses can be used to acquire the same clinical effect (lower dose means lower toxicity to normal tissues and fewer side effects).

The present invention can also be used in the field of cancer research and drug discovery. In this case, the cargo can be a potential medical compound with proapoptotic properties, compound that affects cell cycle or compound that affects cellular viability. The tremendous advantage of the invention is tissue and cell penetration. This allows use of compounds that normally do not penetrate blood-brain barrier and cell membrane.

Another line of application of the invention is to use the tumor selective delivery vector as a tumor labeling machinery, allowing selectively mark or tag all tumor cells (including metastasis). Subsequent tracking of the coupled label permits to use this information for determining the treatment strategy and methods, or use the labeling during the surgery or radiation therapy. We denote such marking entity as a "diagnostic agent". The diagnostic agent can be a contrast agent for the MRI, a radioactive agent, a radiopaque agent, a fluorophore, a fluorescence-quenching system, a marker, a marker enzyme (such as horse-radish peroxidase, beta-galactosidase or other enzyme suitable for tagging the cell), etc.

In accordance with the above, the present invention provides compositions comprising a cell-penetrating peptide and a homing peptide, methods for synthesizing the delivery vector and methods for conjugation of the vector to a cargo.

### Sequence listing free text

pVEC LLIILRRRIRKQAHAHSK-NH₂

gHo NHQQQNPHQPPM-NH₂ (SEQ No.1)

FAM-pVEC LLIILRRRIRKQAHAHSK-NH₂

FAM-gHo NHQQQNPHQPPM-NH₂

FAM-pVEC-gHo (FAM-gHoPe2)
LLIILRRRIRKQAHAHSK-NHQQQNPHQPPM-NH₂

FAM-gHo-pVEC (FAM-gHoPe3)
NHQQQNPHQPPM-LLIILRRRIRKQAHAHSK-NH₂

Dox-pVEC-gHo (Dox-gHoPe2)
XCLLIILRRRIRKQAHAHSK-NHQQQNPHQPPM-NH₂

### Citation list

1. El-Andaloussi, S., T. Holm, and U. Langel, Cell-penetrating peptides: mechanisms and applications. Curr Pharm Des, 2005. 11 (28): p. 3597-611.

2. Elmquist, A., et al., VE-cadherin-derived cell-penetrating peptide, pVEC, with carrier functions. Exp Cell Res, 2001. 269(2): p. 237-44.

3. Lundin, P., et al., Distinct uptake routes of cell-penetrating peptide conjugates. Bioconjug Chem, 2008. 19(12): p. 2535-42.

4. Elmquist, A., M. Hansen, and Langel, Structure-activity relationship study of the cell-penetrating peptide pVEC. Bioch im Biophys Acta, 2006. 1758(6): p. 721-9.

5. US Pat. no. 5527527. Transferrin receptor specific antibody-neuropharmaceutical agent conjugates. Friden, P.

6. US Pat. no. 10077555. Use of cell-penetrating peptides to generate antitumor immunity. Wang, R.

7. US Pat. no. 7431915. Peptides whose uptake by cells is controllable. Jiang, T.

8. US Pat. no. 11133804. Peptides whose uptake by cells is controllable. Jiang, T.

9. US Pat. no. 11141725. Controlled delivery of therapeutic compounds. Neuman, T.

10. US Pat. no. 10400083. HMGN2 peptides and related molecules that selectively home to tumor blood vessels and tumor cells. Ruoslahti, E.

11. Fields, G.B. and R.L. Noble, Solidphase peptide synthesis utilizing 9-fluorenylmethoxycarbonyl amino acids. Int J Pept Protein Res, 1990. 35 (3): p. 161-214.

12. Liang, J.F. and V.C. Yang, Synthesis of doxorubicin-peptide conjugate with multidrug resistant tumor cell killing activity. Bioorg Med Chem Lett, 2005. 15(22): p. 5071-5.

## Claims

1. A system for the targeted delivery of cargo component into human glioma cells, comprising targeting peptide (A) which selectively and efficiently binds human glioma cells, carrier element (B) whereas targeting peptide is attached or linked via linker (L1) covalently to said carrier element, and a cargo component (C) delivered in or onto the cell and attached or linked covalently or noncovalently to the targeting peptide or to the carrier element.

2. The system according to claim 1 wherein targeting peptide is a glioma-homing peptide.

3. The system according to the claim 1 or 2 wherein the targeting peptide is a peptide comprising a nucleic acid sequence according to SEQ ID No 1.

4. The system according to claim 1 wherein the carrier element is a cell-penetrating peptide (CPP), liposome, micelle, dendrimer, carrier polymer, carrier nanoparticle or other carrier system, derived from the ones listed.

5. The system according to claim 1, wherein cargo component is linked via linker (L2) to the carrier element.

6. The system according to claim 3 wherein the cargo component is associated with the carrier element noncovalently via hydrophobic or electrostatic interactions, such as in case of entrapment within the liposome, micelle, dendrimer, nanoparticle.

7. The system according to claim 1 wherein the cargo component is a chemotherapeutic drug, chemotherapeutic agent, apoptotic, cytostatic or cytotoxic entity covalently conjugated to the targeting peptide or carrier element.

8. The system according to claim 1 wherein the cargo component is a chemotherapeutic drug, chemotherapeutic agent, apoptotic, cytostatic or cytotoxic entity attached to the carrier element.

9. The system according to claim 1 wherein the cargo component is a imaging or diagnostic agent for labeling target cells.

10. The system according to claim 5 wherein cargo component is encapsulated within or in the interior of a liposome, micelle, dendrimer, polymer, polyplex or lipoplex.
